(19) Europäisches Patentamt
European Patent Office
Office européen des brevets

(11) EP 0 529 483 B1

(12) **EUROPÄISCHE PATENTSCHRIFT**

(45) Veröffentlichungstag und Bekanntmachung des Hinweises auf die Patenterteilung:
**28.02.1996 Patentblatt 1996/09**

(21) Anmeldenummer: **92114078.6**

(22) Anmeldetag: **18.08.1992**

(51) Int. Cl.$^6$: **C07C 271/22**, C07D 207/273, C07C 229/22, C07C 229/34, C07B 53/00, C12P 17/10 // (C12P17/10, C12R1:72)

(54) **Verfahren zur Herstellung von optisch aktiven 4-Amino-3-hydroxycarbonsäuren**

Process for the preparation of optically active 4-amino-3-hydroxycarboxylic acids

Procédé pour la préparation des acides 4-amino-3-hydroxycarboxyliques optiquement actifs

(84) Benannte Vertragsstaaten:
**AT BE CH DE FR GB IT LI NL SE**

(30) Priorität: **22.08.1991 CH 2471/91**

(43) Veröffentlichungstag der Anmeldung:
**03.03.1993 Patentblatt 1993/09**

(73) Patentinhaber: **LONZA AG**
**CH-3945 Gampel/Wallis (CH)**

(72) Erfinder:
- **Bänziger, Markus, Dr.**
  **Brig-Glis (Kanton Wallis) (CH)**
- **McGarrity, John, Dr.**
  **Visp (Kanton Wallis) (CH)**
- **Meul, Thomas, Dr.**
  **Visp (Kanton Wallis) (CH)**

(74) Vertreter: **Weinhold, Peter, Dr. et al**
**D-80803 München (DE)**

(56) Entgegenhaltungen:
**EP-A- 0 195 717** **EP-A- 0 210 896**
**US-A- 4 876 343**

- **BULLETIN OF THE CHEMICAL SOCIETY OF JAPAN Bd. 49, Nr. 11, 1976, TOKYO JP Seiten 3287 - 3290 T. KATSUKI ET AL. 'The Stereoselective Synthesis of threo-3-Hydroxy-4-amino Acids'**
- **NACHRICHTEN AUS CHEMIE, TECHNIK UND LABORATORIUM Bd. 36, Nr. 7, 1988, WEINHEIM DE Seiten 756 - 758 H.-J. ALTENBACH 'Statin-Synthesen'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 529 483 B1

Printed by Rank Xerox (UK) Business Services
2.9.9/3.4

## Beschreibung

Gegenstand der vorliegenden Erfindung ist ein Verfahren zur Herstellung von optisch aktiven 4-Amino-3-hydroxycarbonsäuren und ihren N-geschützten Derivaten.

Die Produkte des erfindungsgemässen Verfahrens besitzen die allgemeine Formel

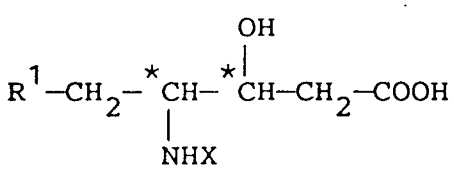

I

worin $R^1$ eine gegebenenfalls verzweigte und/oder substituierte Alkylgruppe mit 1 bis 10 C-Atomen oder eine gegebenenfalls substituierte Aryl-, Arylalkyl- oder Cycloalkylgruppe und X Wasserstoff oder eine Aminoschutzgruppe ist. Diese Verbindungen besitzen zwei Chiralitätszentren und können daher jeweils in 4 stereoisomeren Formen auftreten.

Das erfindungsgemässe Verfahren gestattet es, wahlweise das (3R,4R)- oder das (3S,4S)-Enantiomer herzustellen. Diese beiden Enantiomere werden durch die stereochemischen Bezeichnungen (rel-3R,4R) oder (3R*,4R*) umfasst. Im folgenden wird die erste der beiden Bezeichnungen benutzt.

Einige dieser Verbindungen, insbesondere diejenigen mit $R^1$ = Isopropyl oder Cyclohexyl, sind von Interesse als Baustein von Peptiden, die enzyminhibierende Wirkung aufweisen. Wirksam ist dabei aber nur das (3S,4S)-Stereoisomer. Besonders die unter dem Trivialnamen Statin bekannte (3S,4S)-4-Amino-3-hydroxy-6-methylheptansäure, die in dem Renin-Inhibitor Pepstatin enthalten ist, war bereits Ziel zahlreicher verschiedener Syntheseverfahren (s. z.B. EP 0 210 896; H.J.Altenbach, Nachr.Chem.Tech.Lab. 36, 756-758 (1988); M.Saïah et al, Tetrahedron Asymmetry 2, 111-112 (1991) sowie dort zitierte Literatur). Diese Synthesen eignen sich jedoch nicht oder nur schlecht für die kostengünstige Herstellung grosser Mengen verschieden substituierter 4-Amino-3-hydroxycarbonsäuren, weil sie teils teure Ausgangsmaterialien erfordern, teils nur für den Labormassstab geeignet sind und teils nur für bestimmte Reste $R^1$ der allgemeinen Formel I die benötigten Ausgangsmaterialien überhaupt verfügbar sind.

Aufgabe der vorliegenden Erfindung war daher, ein Verfahren zur Herstellung optisch aktiver (rel-3R,4R)-4-Amino-3-hydroxycarbonsäuren bereitzustellen, das nur preiswerte Ausgangsmaterialien erfordert und in grossem Massstab durchführbar ist.

Erfindungsgemäss wird diese Aufgabe durch das Verfahren nach Patentanspruch 1 gelöst.

Es wurde gefunden, dass die aus 5-Alkylidentetramsäuren der allgemeinen Formel

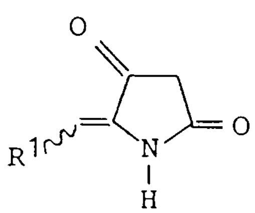

II

bzw. der tautomeren Form

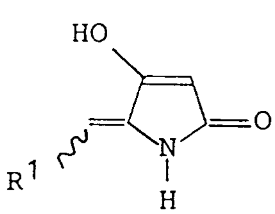

II'

worin $R^1$ die oben genannte Bedeutung hat, und Carbonsäuren bzw. Carbonsäurederivaten der allgemeinen Formel

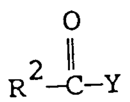

III

worin $R^2$ eine gegebenenfalls verzweigte und/oder substituierte Alkylgruppe mit 1 bis 10 C-Atomen oder eine Arylgruppe und Y Halogen, OH oder $OC(=O)R^2$ ist, erhältlichen 4-O-Acyl-5-alkylidentetramsäuren der allgemeinen Formel

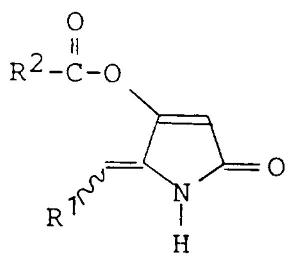

IV

worin $R^1$ und $R^2$ die oben genannte Bedeutung haben, mit guter Stereoselektivität zu einem Gemisch hydriert werden können, welches im wesentlichen aus dem (4RS,5RS)-Racemat der entsprechenden 4-Acyloxy-pyrrolidin-2-one der allgemeinen Formeln

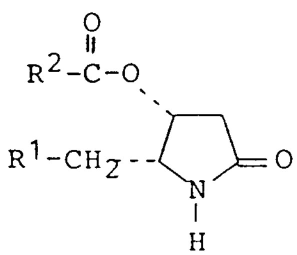

Va und

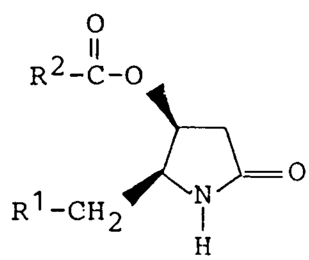

Vb

worin $R^1$ und $R^2$ die oben genannte Bedeutung haben, und geringen Mengen des entsprechenden (4RS,5SR)-Racemats besteht.

Das unerwünschte (4RS,5SR)-Racemat lässt sich leicht abtrennen.

Weiterhin wurde gefunden, dass das Enantiomer mit der (4R,5R)-Konfiguration (Va) in Gegenwart von Lipasen selektiv desacyliert werden kann. Aufgrund des grossen Unterschieds in der Polaritst kann das (4R,5R)-4-Hydroxypyrrolidin-2-on der allgemeinen Formel

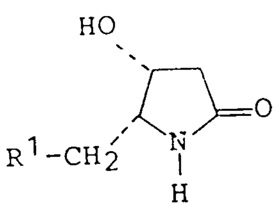

VI

worin $R^1$ die oben genannte Bedeutung hat, leicht abgetrennt werden.

Das unumgesetzte (4S,5S)-4-Acyloxypyrrolidin-2-on (Vb) kann schliesslich analog zu bekannten Verfahren unter Abspaltung der Acylgruppe und Oeffnung des Lactamringes in die entsprechende (3S,4S)-4-Amino-3-hydroxycarbonsäure (I) übergeführt werden.

Alternativ hierzu kann aus dem bei der enzymatischen Desacylierung gebildeten (4R,5R)-4-Hydroxypyrrolidin-2-on (VI) nach dessen Isolierung durch hydrolytische Oeffnung des Lactamrings die entsprechende (3R,4R)-4-Amino-3-hydroxycarbonsäure (I) erhalten werden.

Die für das erfindungsgemässe Verfahren benötigten 5-Alkylidentetramsäuren (II) können nach bekannten Verfahren aus 4-Alkoxy-3-pyrrolin-2-onen und Aldehyden hergestellt werden (EP-Anm. 0 358 128).
Es wurde gefunden, und das stellt keine Ausführungsform der Erfindung dar, dass ihre Synthese wesentlich vereinfacht werden kann, indem die Herstellung von 4-Alkoxy-3-pyrrolin-2-on und die Umsetzung mit dem Aldehyd ohne Isolierung der Zwischenprodukte in einem Topf durchgeführt werden. Zu diesem Zweck wird ein (E)-3-Alkoxy-4-chlorbut-2-ensäurealkylester, der aus 4-Chloracetessigsäurechlorid leicht zugänglich ist (EP-Anm. 0 346 852), mit wässerigem Ammoniak in an sich bekannter Weise (EP 0 216 324) zu dem entsprechenden 4-Alkoxy-3-pyrrolin-2-on cyclisiert und dieses ohne Isolierung in Gegenwart einer starken Base mit dem Aldehyd zu dem entsprechenden 4-Alkoxy-5-alkyliden-3-pyrrolin-2-on kondensiert. Vorzugsweise wird als Ausgangsmaterial der (E)-4-Chlor-3-methoxybut-2-enmethylester und als starke Base Natriumhydroxid verwendet.

Das so erhaltene 4-Alkoxy-5-alkyliden-3-pyrrolin-2-on kann ohne Isolierung auf an sich bekannte Weise durch saure Hydrolyse in die 5-Alkylidentetramsäure (II) übergeführt werden.

Zur Acylierung der 5-Alkylidentetramsäuren (II) zu den 4-Acyloxy-Verbindungen (IV), welche als gemischte Anhydride der entsprechenden Carbonsäuren und Tetramsäuren oder als Enolester aufgefasst werden können, kann grundsätzlich jedes Verfahren zur Herstellung von gemischten Carbonsäureanhydriden oder Carbonsäure-enolestern aus den entsprechenden Carbonsäuren oder Carbonsäurederivaten angewandt werden. Hierzu zählen beispielsweise die Umsetzung mit den Carbonsäuren in Gegenwart von Carbodiimiden oder 1-Acylimidazolen, die Umsetzung mit Carbonsäureanhydriden oder mit Carbonsäurehalogeniden (s. z.B. H.Pielatzik et al. in "Methoden der Organischen Chemie" (Houben-Weyl), 4.Aufl., Bd.E5, S.633-656, Stuttgart 1985, und G.Hesse in "Methoden der Organischen Chemie" (Houben-Weyl), 4.Aufl., Bd. VI/1d, S.108-115, Stuttgart 1978).

Eine bevorzugte Ausführungsform verwendet Carbonsäurehalogenide, insbesondere Carbonsäurechloride, in Gegenwart tertiärer Amine, wie beispielsweise Triethylamin oder Pyridin. Anstelle der freien Tetramsäuren (II) können auch ihre Enolate, beispielsweise die Natriumsalze, eingesetzt werden.

Als Carbonsäuren bzw. Carbonsäurederivate (III) zur Acylierung können aliphatische oder aromatische Carbonsäuren, insbesondere $C_2$-$C_{11}$-Alkansäuren oder Benzoesäuren, bzw. deren Derivate eingesetzt werden, besonders bevorzugt ist Buttersäure bzw. Butyrylchlorid.

Die 4-Acyloxyverbindungen (IV) werden erfindungsgemäss hydriert, wobei zwei Asymmetriezentren gebildet werden. Die Hydrierung verläuft weitgehend stereoselektiv ab, so dass in der Hauptsache das (racemische) (4RS,5RS)-Pyrrolidin-2-on (Va/Vb) neben wenig (4RS,5SR)-Diastereomer entsteht.

Als Katalysator für die Hydrierung wird vorzugsweise ein Palladium-Trägerkatalysator verwendet, beispielsweise 5% Palladium auf Aktivkohle. Anstelle von Palladium kann auch Rhodium, beispielsweise Rhodium auf Aluminiumoxid, oder ein gemischter Pd/Rh-Katalysator eingesetzt werden.

Als Lösungsmittel für die Hydrierung werden vorteilhaft unpolare oder polare aprotische Lösungsmittel eingesetzt, also beispielsweise Toluol oder Essigester oder Tetrahydrofuran.
Die Verwendung protischer Lösungsmittel, wie Alkohole, ist ebenfalls möglich, führt aber zu schlechteren Ausbeuten.

Die Hydrierung wird vorzugsweise etwa bei Raumtemperatur und unter mittlerem Druck von beispielsweise 2 MPa (20 bar) durchgeführt.

Gegebenenfalls bei der Hydrierung entstehende (4RS,5SR)-Pyrrolidin-2-one sowie Hydrogenolysenprodukte können bei der Aufarbeitung ohne weiteres abgetrennt werden, beispielsweise durch einfaches Umkristallisieren, bei dem sie in der Mutterlauge verbleiben.

Das Racemat aus dem (4R,5R)- und dem (4S,5S)-Pyrrolidin-2-on (Va/Vb) wird erfindungsgemäss mit einer Lipase enantioselektiv desacyliert. Als Lipase wird vorzugsweise eine Lipase aus Candida cylindracea eingesetzt. Diese Lipase hydrolysiert enantioselektiv das (4R,5R)-4-Acyloxypyrrolidin-2-on (Va) zur entsprechenden 4-Hydroxy-Verbindung.

Diese Desacylierung wird vorteilhaft in wässerigem Medium bei einem pH in der Nähe des Neutralpunkts, vorzugsweise bei pH 6-8, durchgeführt, um eine nichtenzymatische Desacylierung bzw. Hydrolyse zu unterdrücken.
Die Temperatur liegt zweckmässig bei 0 bis 40°C, vorzugsweise bei 0 bis 25°C.

Während der Reaktion wird der pH vorzugsweise von einer geeigneten Regeleinrichtung ("pH-Stat") durch Zudosieren einer starken Base konstant gehalten.
Als starke Base wird vorzugsweise Natronlauge eingesetzt.
Anhand der zudosierten Basenmenge lässt sich der Reaktionsumsatz bestimmen. Zweckmässig wird die Reaktion nach einem Umsatz von ca. 50% der gesamten eingesetzten Menge (Va+Vb) abgebrochen. Je nachdem, ob das nicht hydrolysierte oder das hydrolysierte Enantiomer weiterverarbeitet werden soll, wird die Reaktion vorteilhaft bis zu einem Umsatz von wenig mehr als 50% bzw. etwas weniger als 50% durchgeführt, um ein Produkt mit möglichst hoher optischer Reinheit zu erhalten.

Die Abtrennung der Hydroxy-Verbindung (VI) von der Acyloxy-Verbindung (Vb) wird vorteilhaft durch Extraktion mit einem Lösungsmittel geringer Polarität, beispielsweise Toluol, durchgeführt. Die Hydroxy-Verbindung bleibt dabei in der wässerigen Phase, während die Acyloxy-Verbindung in die organische Phase übergeht.

Die erfindungsgemäss hergestellte optisch aktive Acyloxy-Verbindung bzw. Hydroxy-Verbindung wird schliesslich analog zu bekannten Verfahren (EP 0 210 896) durch Hydrolyse des Lactamrings und gegebenenfalls der Esterfunktion in die Zielverbindung übergeführt. Für die saure Hydrolyse wird vorzugsweise eine Säure als Katalysator eingesetzt, besonders bevorzugt ist Bromwasserstoffsäure.

Die ungeschützten 4-Amino-3-hydroxycarbonsäuren neigen zu spontaner Lactambildung und werden daher vorteilhaft an der Aminogruppe mit einer Schutzgruppe versehen. Solche Schutzgruppen und die Methoden zu ihrer Einführung und Entfernung sind dem Fachmann bekannt, als Beispiel soll hier nur die tert-Butoxycarbonyl-Gruppe genannt werden.
Anstelle der Aminogruppe kann auch die Carboxylgruppe geschützt werden, beispielsweise durch Veresterung.

Die nachfolgenden Beispiele verdeutlichen die Durchführung des erfindungsgemässen Verfahrens.

Referenzbeispiel 1

(Z)-5-Isobutyliden-4-methoxy-3-pyrrolin-2-on

Zu 200 ml konzentrierter wässeriger Ammoniaklösung wurden bei 65-70°C unter ständigem Durchleiten von Ammoniakgas innerhalb von 3 h 120 g (E)-2-Chlor-3-methoxybut-2-ensäuremethylester getropft. Das Reaktionsgemisch wurde weitere 1,5 h bei dieser Temperatur gerührt und dann noch 0,5 h zum Rückfluss erhitzt.
Nach dem Abkühlen auf Raumtemperatur wurden 450 ml 1M Natronlauge zugegeben und durch weiteren Zusatz von 33%iger Natronlauge der pH auf >13 gebracht.
Anschliessend wurden 50 g Isobutyraldehyd zugesetzt und das Reaktionsgemisch unter Rühren 6 h auf 60°C erhitzt.
Nach Abkühlen auf Raumtemperatur wurde das ausgefallene Produkt abfiltriert, mit kaltem Wasser gewaschen und im Vakuumtrockenschrank getrocknet.
Ausbeute: 93,2 g (80% d.Th.)
Schmp.: 140,5-141,1°C (aus Diethylether)

Referenzbeispiel 2

(Z)-5-Isobutylidenpyrrolidin-2,4-dion (II, $R^1$ = Isopropyl)

50 g fein gepulvertes (Z)-5-Isobutyliden-4-methoxy-3-pyrrolin-2-on wurden mit 500 ml konzentrierter Salzsäure 5 h bei Raumtemperatur gerührt. Das Gemisch wurde anschliessend 5 h bei Raumtemperatur gerührt und langsam mit 1 l 14%iger Natronlauge versetzt.
Das ausgefallene gelbe Produkt wurde abfiltriert, mit kaltem Wasser gewaschen und im Vakuumtrockenschrank getrocknet.
Ausbeute: 42,0 g (92% d.Th.)
Schmp.: 134-136°C (THF/Hexan)

Referenzbeispiel 3

(Z)-4-Butyryloxy-5-isobutyliden-3-pyrrolin-2-on (IV, $R^1$ = Isopropyl, $R^2$ = Propyl)

In 318 ml Dichlormethan wurden 31,8 g (Z)-5-Isobutylidenpyrrolidin-2,4-dion aufgeschlämmt und auf -5°C abgekühlt. Anschliessend wurden zunächst 23,22 g Butyrylchlorid und danach 27,32 g Triethylamin zugesetzt. Nach der Zugabe wurde das Gemisch noch 10 min bei 0°C gerührt, anschliessend mit 100 ml Dichlormethan verdünnt, zweimal mit je 100 ml 5%iger $NaHCO_3$-Lösung und zweimal mit je 100 ml 0,4 M Salzsäure gewaschen.
Die wässerigen Phasen wurden getrennt mit Dichlormethan extrahiert und die vereinigten organischen Phasen über Magnesiumsulfat getrocknet.
Nach Abdestillieren des Lösungsmittels wurde das Rohprodukt (46 g) aus Hexan umkristallisiert.
Ausbeute: 38,8 g (84% d.Th.)
Schmp.: 111-112°C

$^1H$ NMR ($CDCl_3$; 400 MHz):
1,03 (t, J=7,5, 3H)
1,12 (d, J=6,6, 6H)
1,77 (m, 2H)
2,56 (t, J=7,5, 2H)

2,78-2,83 (m, 1H)
5,35 (d, J=10, 1H)
6,15 (br.s, 1H)
9,91 (br.s, 1H)

Beispiel 1

(4RS,5RS)-4-Butyryloxy-5-isobutylpyrrolidin-2-on (Va/Vb, $R^1$ = Isopropyl, $R^2$ = Propyl)

In 300 ml Toluol wurden 30,0 g (Z)-4-Butyryloxy-5-isobutyliden-3-pyrrolin-2-on (hergestellt nach Referenzbeispiel 3) gelöst, mit 3,0 g Palladium/Aktivkohle (5% Pd) versetzt und im Autoklaven bei 2 MPa (20 bar) 24 h hydriert. Anschliessend wurde der Katalysator abfiltriert, das Filtrat eingeengt und der Rückstand aus Hexan umkristallisiert.
Ausbeute: 23,50 g (77% d.Th.)
Schmp.: 101,5-102,7°C

$^1$H-NMR ($CDCl_3$; 400 MHz): 0,91-0,98 (m, 9H)
1,35-1,71 (m, 5H)
2,29-2,36 (m, 3H)
2,71 (dd, J=6,4, 17,6, 1H)
3,88-3,93 (m, 1H)
5,40-5,43 (m, 1H)
7,49 (br.s, 1H)

Beispiel 2

(4S,5S)-4-Butyryloxy-5-isobutylpyrrolidin-2-on (Vb, $R^1$ = Isopropyl, $R^2$ = Propyl)

In 100 ml Wasser wurden 20 g (4RS,5RS)-4-Butyryloxy-5-isobutylpyrrolidin-2-on (hergestellt nach Beispiel 2) und 2 g Candida cylindracea-Lipase (Biocatalysts) bei Raumtemperatur gerührt.
Durch Zudosieren von 1M Natronlauge wurde der pH der Suspension konstant bei 7 gehalten. Nach 70 h und einem Verbrauch von 47,36 ml 1M Natronlauge (entsprechend 53,8% Umsatz) wurde die Reaktion abgebrochen.

Das Reaktionsgemisch wurde mit 200 ml Wasser verdünnt und einmal mit 400 ml und noch zweimal mit je 200 ml Toluol extrahiert. Die vereinigten Toluolphasen wurden im Vakuum eingeengt und der Rückstand im Hochvakuum getrocknet.
Ausbeute: 8,55 g (85,5%, bezogen auf ein Enantiomer)
Schmp.: 94,9-95,3°C
$[\alpha]_D^{20}$: -3,9° (c = 1,0; $CHCl_3$)
ee-Wert (GC, Lipodex$^R$ D-Säule) >99%

Beispiel 3

(3S,4S)-4-(tert-Butoxycarbonylamino)-3-hydroxy-6-methylheptansäure (Boc-Statin) (I, $R^1$ = Isopropyl, X = tert-Butoxycarbonyl)

3,0 g (4S,5S)-4-Butyryloxy-5-isobutylpyrrolidin-2-on (hergestellt nach Beispiel 2) wurden in 30 ml 48%iger wässriger Bromwasserstoffsäure 24 h bei 75°C gerührt. Nach dem Abkühlen auf Raumtemperatur wurde das Gemisch mit 30 ml Wasser verdünnt und zur Entfernung der Buttersäure dreimal mit je 20 ml Diethylether extrahiert.
Die wässrige Phase wurde auf -10°C abgekühlt und mit 33%iger Natronlauge auf pH 10 eingestellt. Anschliessend wurden 20 ml Tetrahydrofuran und 2,9 g Di-tert-butyldicarbonat zugegeben.
Man liess das Gemisch 94 h bei Raumtemperatur reagieren, wobei der pH durch Zugabe von 1M NaOH konstant gehalten wurde.
Anschliessend wurde das Gemisch mit 16%iger Salzsäure auf pH 1,4 angesäuert und rasch dreimal mit je 100 ml Diethylether extrahiert. Die Etherphase wurde über Magnesiumsulfat getrocknet und im Vakuum eingeengt. Der Rückstand wurde durch Säulenchromatographie an Kieselgel (Hexan/Ethylacetat/Essigsäure 6:4:0,1) gereinigt.
Ausbeute: 2,09 g (58% d.Th.)
Schmp.: 120,2-120,9°C (Aceton/Hexan)
$[\alpha]_D^{20}$: -40,3° (c = 1,0; $CH_3OH$)

$^1H$-NMR ($d_6$-DMSO; 400 MHz):
0,83-0,88 (m, 6H)
1,22-1,29 (m, 2H)
1,38 (s, 9H)
1,53-1,57 (m, 1H)
2,12 (dd, J=15,5, 9,1, 1H)
2,34 (dd, J=15,5, 3,8, 1H)
3,49-3,53 (m, 1H)
3,79-3,82 (m, 1H)
6,24 (d, J=9,1, 1H)

Referenzbeispiel 4

(Z)-5-Benzylidenpyrrolidin-2,4-dion (II, $R^1$ = Phenyl)

133 g konzentrierte wässerige Ammoniaklösung wurden auf 65-70°C erhitzt. Unter ständigem Durchleiten von Ammoniakgas wurden innerhalb von 3 h 75 g (E)-2-Chlor-3-methoxybut-2-ensäuremethylester zugetropft. Das Gemisch wurde noch 45 min bei dieser Temperatur gerührt, 30 min auf Rückflusstemperatur erhitzt und anschliessend auf Raumtemperatur abgekühlt.
Nach Zugabe von 300 ml Wasser und 50 ml 33%iger Natronlauge wurden 46,4 g Benzaldehyd zugesetzt. Das Gemisch wurde 6 h unter Rühren auf 60°C erhitzt und anschliessend auf Raumtemperatur abgekühlt. Danach wurden 688 ml konzentrierte Salzsäure zugegeben und das Reaktionsgemisch bei 40°C 22 h gerührt.
Nach Abkühlen auf Raumtemperatur wurde das gelbe Produkt abfiltriert, mit kaltem Wasser gewaschen und im Vakuumtrockenschrank getrocknet.
Ausbeute: 74,66 g (88% d.Th.)
Schmp.: 186-187°C (THF/Hexan)

Referenzbeispiel 5

(Z)-5-Benzyliden-4-butyryloxy-3-pyrrolin-2-on (IV, $R^1$ = Phenyl, $R^2$ = Propyl)

Zu einer auf 0°C gekühlten Suspension von 67,0 g (Z)-5-Benzylidenpyrrolidin-2,4-dion in 670 ml Dichlormethan wurden 40,0 g Butyrylchlorid und anschliessend 47,1 g Triethylamin gegeben und das Gemisch noch 5 min bei 0°C gerührt. Anschliessend wurde das Gemisch zweimal mit je 200 ml 5%iger $NaHCO_3$-Lösung und dann zweimal mit je 200 ml 0,5M Salzsäure gewaschen. Die Wasserphasen wurden getrennt mit Dichlormethan extrahiert. Die vereinigten organischen Phasen wurden über Magnesiumsulfat getrocknet und im Vakuum eingeengt. Der Rückstand wurde aus 230 ml Toluol umkristallisiert.
Ausbeute: 67,25 g (73% d.Th.)
Schmp.: 139,8-141,5°C

$^1H$-NMR ($CDCl_3$; 400 MHz):
1,03 (t, J=7,4, 3H)
1,78 (m, 2H)
2,58 (t, J=6,7, 2H)
6,20 (s, 1H)
6,30 (s, 1H)
7,28-7,50 (m, 5H)
9,20 (br.s, 1H)

Beispiel 4

(4RS,5RS)-5-Benzyl-4-butyryloxypyrrolidin-2-on (Va/Vb, $R^1$ = Phenyl, $R^2$ = Propyl)

In 467 ml Toluol wurden 46,66 g (Z)-5-Benzyliden-4-butyryloxy-3-pyrrolin-2-on (hergestellt nach Referenzbeispiel 5) aufgeschlämmt und mit 4,67 g Palladium/Aktivkohle (5% Pd) im Autoklaven bei Raumtemperatur und 2 MPa (20 bar) 28 h hydriert. Anschliessend wurde der Katalysator abfiltriert und das Filtrat im Vakuum eingeengt. Der Rückstand wurde aus Diisopropylether umkristallisiert.
Ausbeute: 32,88 g (69% d.Th.)
Schmp.: 85,6-87°C

$^1H$-NMR ($CDCl_3$, 400 MHz): 0,98 (t, J=7,3, 3H)
1,69 (sext., J=7,3, 2H)
2,34-2,41 (m, 3H)
2,68-2,77 (m, 2H)
2,92 (dd, J=14,0, 5,1, 1H)
4,07-4,18 (m, 1H)
5,40-5,48 (m, 1H)
6,04 (br.s, 1H)
7,16-7,34 (m, 5H)

Beispiel 5

(4S,5S)-5-Benzyl-4-butyryloxypyrrolidin-2-on (Vb, $R^1$ = Phenyl, $R^2$ = Propyl)

In einem zweiphasigen System aus 145 ml Wasser und 36 ml Toluol wurden 21,14 g (4RS,5RS)-5-Benzyl-4-butyryloxypyrrolidin-2-on (hergestellt nach Beispiel 4) und 4,22 g Candida cylindracea-Lipase (Biocatalysts) analog zu Beispiel 2 umgesetzt. Nach 116 h und dem Verbrauch von 47,01 ml 1M Natronlauge (entsprechend 58% Umsatz) wurde die Reaktion abgebrochen.
Das Reaktionsgemisch wurde mit 600 ml Toluol und 250 ml Wasser verdünnt und 30 min kräftig durchgerührt. Die Phasen wurden getrennt und die Wasserphase mit 300 ml Toluol extrahiert.
Die vereinigten Toluolphasen wurden im Vakuum eingeengt, der Rückstand in 170 ml Toluol aufgenommen, dreimal mit je 40 ml Wasser gewaschen und nochmals im Vakuum eingedampft.
Das so erhaltene Rohprodukt wurde aus Diisopropylether umkristallisiert.
Ausbeute: 7,80 g (74%, bezogen auf ein Enantiomer)
Schmp.: 78,9-79,2°C
$[\alpha]_D^{20}$: -89,0° (c = 1,0; $CHCl_3$)
ee-Wert: >99,9% (HPLC, Chiralcel$^R$-OD-Säule)

Beispiel 6

(3S,4S)-4-(tert-Butoxycarbonylamino)-3-hydroxy-5-phenylpentansäure (I, $R^1$ = Phenyl, X = tert-Butoxycarbonyl)

In 10 ml 48%iger wässeriger Bromwasserstoffsäure wurde 1,0 g (4S,5S)-5-Benzyl-4-butyryloxypyrrolidin-2-on (hergestellt nach Beispiel 5) 20 h bei 80°C gerührt. Das Gemisch wurde anschliessend auf Raumtemperatur abgekühlt, mit 10 ml Wasser verdünnt und zur Entfernung der Buttersäure dreimal mit je 20 ml Diethylether extrahiert. Die Etherphase wurde verworfen.
Die Wasserphase wurde auf -5°C abgekühlt und mit 33%iger Natronlauge auf pH 10 eingestellt. Dann wurden 20 ml Tetrahydrofuran und 0,86 g Di-tert-butyldicarbonat zugesetzt und das Gemisch bei Raumtemperatur und konstantem pH 10 24 h zur Reaktion gebracht.
Anschliessend wurde das Gemisch mit 1M Salzsäure auf pH 1 angesäuert und dreimal mit je 50 ml Diethylether extrahiert.
Die vereinigten Etherphasen wurden über Magnesiumsulfat getrocknet und im Vakuum eingeengt.
Der Rückstand wurde durch Säulenchromatographie an Kieselgel (Hexan/Ethylacetat/Essigsäure 10:10:0,25) gereinigt.
Ausbeute: 0,64 g (54% d.Th.)
Schmp.: 153,2-153,4°C ($CHCl_3$/Hexan)
$[\alpha]_D^{20}$: -37,7° (c = 1,1; $CH_3OH$)

$^1H$-NMR ($CDCl_3$, 60°C; 400 MHz): 1,40 (s, 9H)
2,40-2,66 (m, 2H)
2,86-2,96 (m, 2H)
3,66-3,82 (m, 1H)
3,96-4,07 (m, 1H)
4,82-5,00 (br.s, 1H)
7,12-7,35 (m, 5H)

Beispiel 7

(4S,5S)-4-Butyryloxy-5-(cyclohexylmethyl)pyrrolidin-2-on (Vb, $R^1$ = Cyclohexyl, $R^2$ = Propyl)

In 10 ml Ethylacetat wurden 1,0 g (4S,5S)-5-Benzyl-4-butyryloxypyrrolidin-2-on (hergestellt nach Beispiel 5) gelöst, mit 100 mg Rhodium/Aktivkohle (5% Rh, Johnson Matthey Typ 20A) versetzt und im Autoklaven bei Raumtemperatur bei 2 MPa (20 bar) 20 h hydriert.
Anschliessend wurde der Katalysator abfiltriert und das Filtrat im Vakuum eingedampft.
Ausbeute: 1,0 g zähes Oel (ca. 98%)

$^1$H-NMR ($CDCl_3$; 300 MHz): 0,8-1,35 (m, 9H)
1,45 (t, 2H)
1,58-1,80 (m, 7H)
2,22-2,40 (m, 3H)
2,70 (dd, 1H)
3,85-3,98 (m, 1H)
5,35-5,45 (m, 1H)
6,35 (br.s, 1H)

## Patentansprüche

1. Verfahren zur Herstellung von optisch aktiven (rel-3R,4R)-4-Amino-3-hydroxycarbonsäuren der allgemeinen Formel

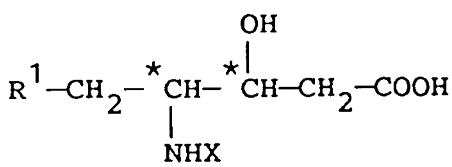

I

worin $R^1$ eine gegebenenfalls verzweigte und/oder substituierte Alkylgruppe mit 1 bis 10 C-Atomen oder eine gegebenenfalls substituierte Aryl-, Arylalkyl- oder Cycloalkylgruppe und X Wasserstoff oder eine Aminoschutzgruppe bedeutet, dadurch gekennzeichnet, dass eine substituierte Tetramsäure der allgemeinen Formel

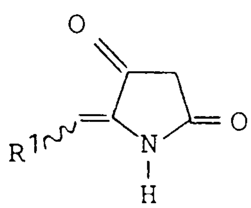

II

worin $R^1$ die oben genannte Bedeutung hat, mit einer Carbonsäure oder einem Carbonsäurederivat der allgemeinen Formel

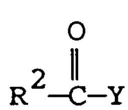

III

worin $R^2$ eine gegebenenfalls verzweigte und/oder substituierte Alkylgruppe mit 1 bis 10 C-Atomen oder eine Arylgruppe und Y Halogen, OH oder $OC(=O)R^2$ ist, zu einer Verbindung der allgemeinen Formel

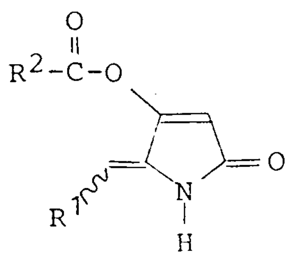

IV

acyliert, anschliessend stereoselektiv zu den entsprechenden enantiomeren Pyrrolidin-2-onen der allgemeinen Formel

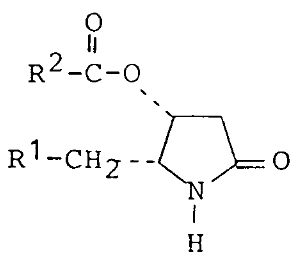

Va und

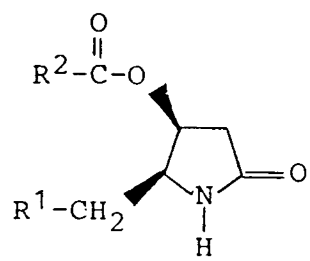

Vb

hydriert, das (4R,5R)-Enantiomer (Va) mit einer Lipase enantioselektiv zu dem entsprechenden (4R,5R)-4-Hydroxypyrrolidin-2-on der allgemeinen Formel

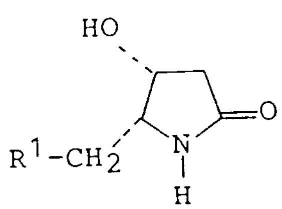

VI

hydrolysiert und von dem nicht hydrolysierten (4S,5S)-Enantiomer (Vb) abgetrennt und anschliessend wahlweise die Acyloxy-Verbindung Vb oder die Hydroxy-Verbindung VI unter Spaltung des Lactam-Rings zur Zielverbindung I (X = H) hydrolysiert und gegebenenfalls nach bekannten Verfahren in die entsprechende N-geschützte Form übergeführt wird.

2. Verfahren nach Patentanspruch 1, dadurch gekennzeichnet, dass als Carbonsäure oder Carbonsäurederivat (III) Buttersäure oder ein Derivat der Buttersäure eingesetzt wird.

3. Verfahren nach Patentanspruch 2, dadurch gekennzeichnet, dass als Buttersäurederivat Butyrylchlorid eingesetzt wird.

4. Verfahren nach einem der Patentansprüche 1 bis 3, dadurch gekennzeichnet, dass die stereoselektive Hydrierung mit einem Katalysator aus der Gruppe der Palladium- oder Rhodiumkatalysatoren oder der Palladium/Rhodium-Mischkatalysatoren durchgeführt wird.

5. Verfahren nach Patentanspruch 4, dadurch gekennzeichnet, dass als Katalysator ein Palladium-Trägerkatalysator eingesetzt wird.

6. Verfahren nach einem der Patentansprüche 1 bis 5, dadurch gekennzeichnet, dass als Lipase eine Lipase aus Candida cylindracea eingesetzt wird.

7. Verfahren nach einem der Patentansprüche 1 bis 6, dadurch gekennzeichnet, dass nach der enantioselektiven Hydrolyse das nicht hydrolysierte (4S,5S)-Enantiomer (Vb) zur Zielverbindung umgesetzt wird.

8. Verfahren nach einem der Patentansprüche 1 bis 7, dadurch gekennzeichnet, dass nach der Spaltung des Lactam-Rings die tert-Butoxycarbonylgruppe als Aminoschutzgruppe X eingeführt wird.

9. Verfahren nach einem der Patentansprüche 1 bis 8, dadurch gekennzeichnet, dass als substituierte Tetramsäure (II) eine 5-Isobutyliden- ($R^1$ = Isopropyl) oder 5-Benzylidentetramsäure ($R^1$ = Phenyl) eingesetzt wird.

10. (4S,5S)-4-Acyloxypyrrolidin-2-one der allgemeinen Formel

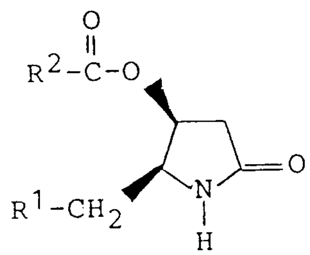

Vb

worin $R^1$ eine gegebenenfalls verzweigte und/oder substituierte Alkylgruppe mit 1 bis 10 C-Atomen oder eine gegebenenfalls substituierte Aryl-, Arylalkyl- oder Cycloalkylgruppe und $R^2$ eine gegebenenfalls verzweigte und/oder substituierte Alkylgruppe mit 1 bis 10 C-Atomen oder eine gegebenenfalls substituierte Arylgruppe ist.

11. Verbindung nach Patentanspruch 10, worin $R^1$ Isopropyl, Phenyl oder Cyclohexyl ist.

12. Verbindung nach Patentanspruch 10 oder 11, worin $R^2$ Propyl ist.

## Claims

1. A process for the production of optically active (rel-3R,4R)-4-amino-3-hydroxycarboxylic acids of the general formula:

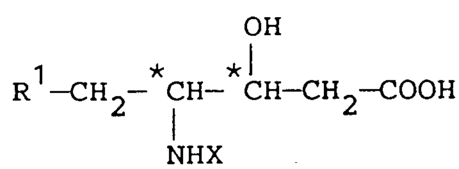

I

wherein $R^1$ is an optionally branched and/or substituted alkyl group having 1 to 10 C atoms or an optionally substituted aryl, arylalkyl or cycloalkyl group, and X is hydrogen or an amino-protective group, characterized in that a substituted tetramic acid of the general formula:

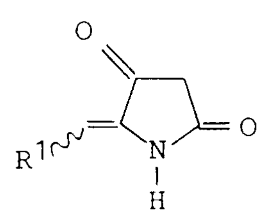

II

wherein $R^1$ has the above-mentioned meaning, is acylated with a carboxylic acid or a carboxylic acid derivative of

the general formula:

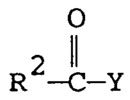

III

wherein $R^2$ is an optionally branched and/or substituted alkyl group having 1 to 10 C atoms or an aryl group, and Y is halogen, OH or $OC(=O)R^2$, to a compound of the general formula:

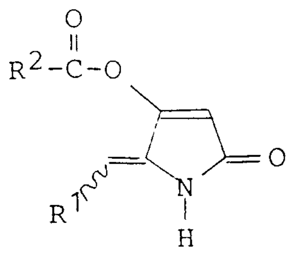

IV

which is then stereoselectively hydrogenated to the corresponding enantiomeric pyrrolidin-2-ones of the general formulas:

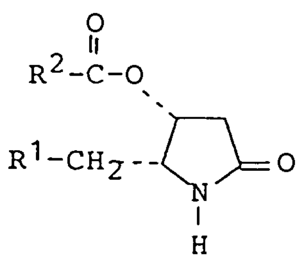

Va and

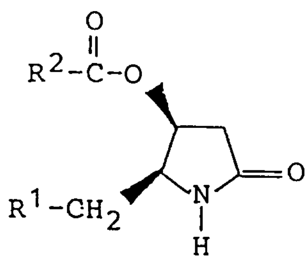

Vb

and the (4R,5R)-enantiomer (Va) is enantioselectively hydrolyzed with a lipase to the corresponding (4R,5R)-4-hydroxypyrrolidin-2-one of the general formula:

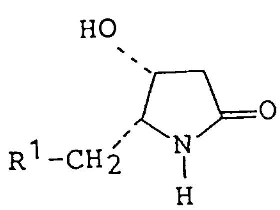

VI

and separated from the nonhydrolyzed (4S,5S)-enantiomer (Vb), and then the acyloxy compound Vb or, alternatively, the hydroxy compound VI is hydrolyzed, with cleavage of the lactam ring, to the target compound I (X = H), and optionally converted, according to known procedures, to the corresponding N-protected form.

2. The process according to claim 1, characterized in that butyric acid or a butyric acid derivative is used as the carboxylic acid or carboxylic acid derivative (III).

3. The process according to claim 2, characterized in that butyryl chloride is used as the butyric acid derivative.

4. The process according to one of the claims 1 to 3, characterized in that the stereoselective hydrogenation is performed with a catalyst from the group of palladium or rhodium catalysts or palladium/rhodium mixed catalysts.

5. The process according to claim 4, characterized in that as the catalyst a supported palladium catalyst is used.

6. The process according to one of the claims 1 to 5, characterized in that as the lipase a lipase from Candida cylindracea is used.

7. The process according to one of the claims 1 to 6, characterized in that, after the enantioselective hydrolysis, the nonhydrolyzed (4S,5S)-enantiomer (Vb) is reacted to the target compound.

8. The process according to one of the claims 1 to 7, characterized in that, after the cleavage of the lactam ring, a tert-butoxycarbonyl group is introduced as the amino-protective group X.

9. The process according to one of the claims 1 to 8, characterized in that a 5-isobutylidene tetramic acid ($R^1$ = isopropyl) or 5-benzylidene tetramic acid ($R^1$ = phenyl) is used as the substituted tetramic acid (II).

10. (4S,5S)-4-Acyloxypyrrolidin-2-ones of the general formula:

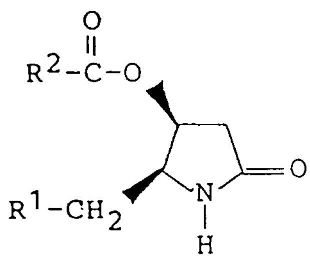

Vb

wherein $R^1$ is an optionally branched and/or substituted alkyl group having 1 to 10 C atoms or an optionally substituted aryl, arylalkyl or cycloalkyl group, and $R^2$ is an optionally branched and/or substituted alkyl group having 1 to 10 C atoms or an optionally substituted aryl group.

11. The compound according to claim 10, wherein $R^1$ is isopropyl, phenyl or cyclohexyl.

12. The compound according to claim 10 or 11, wherein $R^2$ is propyl.

**Revendications**

1. Procédé pour la préparation d'acides (rel-3R, 4R)-4-amino-3-hydroxycarboxyliques, optiquement actifs, de formule générale

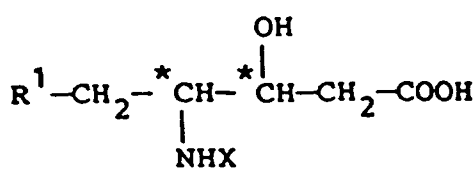

I

dans laquelle $R^1$ signifie un groupe alkyle éventuellement ramifié et/ou substitué avec 1 jusqu'à 10 atomes C ou un groupe aryle, arylalkyle ou cycloalkyle éventuellement substitué et X signifie hydrogène ou un groupe protecteur

amino, caractérisé en ce que l'on acyle un acide tétramique substitué de formule générale

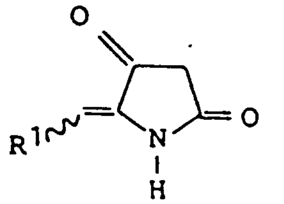

II

dans laquelle $R^1$ à la signification précitée, avec un acide carboxylique ou un dérivé de l'acide carboxylique de formule générale

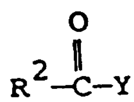

III

dans laquelle $R^2$ est éventuellement un groupe alkyle ramifié et/ou substitué avec 1 à 10 atomes C ou un groupe aryle et Y est l'halogène, OH ou $OC(=O)R^2$, en un composé de formule générale

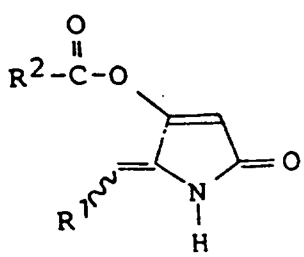

IV

puis on hydrogène stéréosélectivement en les pyrrolidine-2-ones énantiomères correspondantes de formule générale

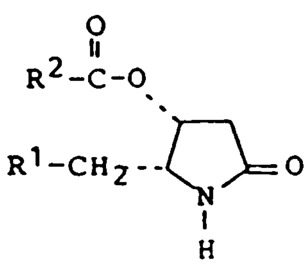

Va und

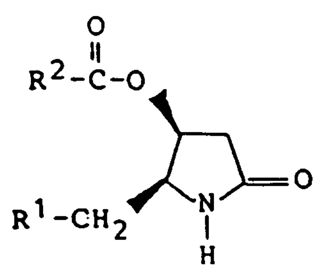

Vb

on hydrolyse le (4R, 5R)-énantiomère (Va) avec une lipase énantiosélectivement en la (4R, 5R)-4-hydroxypyrrolidine-2-one correspondante de la formule générale

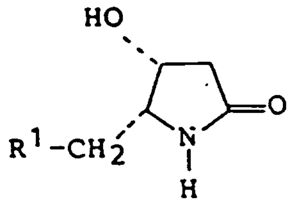

VI

et on sépare du (4S, 5S)-énantiomère (Vb) non hydrolysé et consécutivement, on hydrolyse au choix le composé

acyloxy Vb ou le composé hydroxy VI par clivage du cycle lactame en le composé cible I (X = H) et on transforme éventuellement selon un procédé connu en la forme correspondante N protégée.

2. Procédé selon la revendication 1, caractérisé en ce qu'en tant qu'acide carboxylique ou de dérivé d'acide carboxylique (III) on met en oeuvre de l'acide butyrique ou un dérivé de l'acide butyrique.

3. Procédé selon la revendication 2, caractérisé en ce qu'en tant que dérivé de l'acide butyrique on met en oeuvre du chlorure de butyryle.

4. Procédé selon l'une des revendications 1 à 3, caractérisé en ce que l'hydrogénation stéréosélective est conduite avec un catalyseur du groupe des catalyseurs palladium ou rhodium ou des catalyseurs mixtes palladium/ rhodium.

5. Procédé selon la revendication 4, caractérisé en ce qu'en tant que catalyseur, on met en oeuvre un catalyseur porteur de palladium.

6. Procédé selon l'une des revendications 1 à 5, caractérisé en ce qu'en tant que lipase, on met en oeuvre une lipase de Candida cylindracea.

7. Procédé selon l'une des revendications 1 à 6, caractérisé en ce qu'après l'hydrolyse énantiosélective on transforme le (45, 5S)-énantiomère Vb non hydrolysé en le composé cible.

8. Procédé selon l'une des revendications 1 à 7, caractérisé en ce qu'après le clivage du cycle lactame on introduit le groupe tert-butoxycarbonyle en tant que groupe amino protecteur X.

9. Procédé selon l'une des revendications 1 à 8, caractérisé en ce qu'en tant qu'acide tétramique substitué II, on met en oeuvre un acide 5-isobutyliden-($R^1$ = isopropyle) ou 5-benzylidène tétramique ($R^1$ = phényle).

10. (4S, 5S)-4-acyloxypyrrolidin-2-ones de formule générale

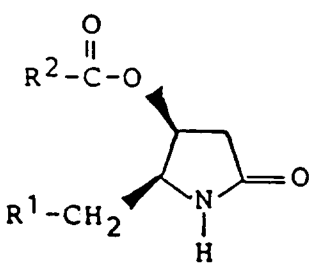

Vb

dans laquelle $R^1$ est éventuellement un groupe alkyle ramifié et/ou substitué avec 1 à 10 atomes de carbone ou un groupe aryle, arylalkyle ou cycloalkyle éventuellement substitué et $R^2$ est un groupe alkyle éventuellement ramifié et/ou substitué avec 1 à 10 atomes de carbone ou un groupe aryle éventuellement substitué.

11. Composé selon la revendication 10, dans lequel $R^1$ est isopropyle, phényle ou cyclohéxyle.

12. Composé selon la revendication 10 ou 11, dans lequel $R^2$ est propyle.